# EUROPÄISCHE PATENTSCHRIFT

(11) **EP 1 874 764 B1**
(45) Veröffentlichungstag und Bekanntmachung des Hinweises auf die Patenterteilung: **10.11.2010**
(21) Anmeldenummer: 06724168.7
(22) Anmeldetag: 08.04.2006
(51) Int. Cl.: C07D 413/14, C07D 413/10, C07D 413/12, C07D 263/28, A61K 31/5377, A61K 31/421, A61P 7/02

(54) **IMINO-OXAZOLIDINE UND IHRE VERWENDUNG ALS ANTIKOAGULANTIEN**
IMINO-OXAZOLIDINES AND USE THEREOF AS ANTICOAGULANTS
IMINO-OXAZOLIDINES ET LEUR UTILISATION

(30) Priorität: 22.04.2005 DE 102005018690
(43) Veröffentlichungstag der Anmeldung: 09.01.2008
(73) Patentinhaber: Bayer Schering Pharma Aktiengesellschaft, 13353 Berlin (DE)
(72) Erfinder: RÖHRIG, Susanne, 45276 Essen (DE); POHLMANN, Jens, CH-4058 Basel (CH); PERZBORN, Elisabeth, 42327 Wuppertal (DE); GERDES, Christoph, 51373 Leverkusen (DE); SCHLEMMER, Karl-Heinz, 42113 Wuppertal (DE)
(86) Internationale Anmeldenummer: PCT/EP2006/003232
(87) Internationale Veröffentlichungsnummer: WO 2006/111285

(56) Entgegenhaltungen:
- WO-A-01/47919

## Beschreibung

Die vorliegende Anmeldung betrifft neue Imino-oxazolidine, Verfahren zu ihrer Herstellung, ihre Verwendung zur Herstellung von Arzneimitteln zur Behandlung und/oder Prophylaxe von Krankheiten, insbesondere von thromboembolischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden bzw. minimiert werden. Die Blutstillung nach Gefäßverletzung erfolgt im wesentlichen durch das Gerinnungssystem, bei dem eine enzymatische Kaskade komplexer Reaktionen von Plasmaproteinen ausgelöst wird. Hieran sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommt dem Faktor Xa, der aus dem Proenzym Faktor X gebildet wird, eine Schlüsselrolle zu, da er beide Gerinnungswege verbindet. Die aktivierte Serinprotease Xa spaltet Prothrombin zu Thrombin. Das entstandene Thrombin wiederum spaltet seinerseits Fibrinogen zu Fibrin. Durch anschließende Quervernetzung der Fibrin-Monomere kommt es zur Bildung von Blutgerinnseln und damit zur Blutstillung. Darüber hinaus ist Thrombin ein potenter Auslöser der Thrombozytenaggregation, die ebenfalls einen erheblichen Beitrag bei der Hämostase leistet.

Die Hämostase unterliegt einem komplexen Regulationsmechanismus. Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Dies kann zu schwerwiegenden thromboembolischen Erkrankungen führen. Darüber hinaus kann eine Hyperkoagulabilität - systemisch - bei einer Verbrauchskoagulopathie zur disseminierten intravasalen Gerinnung führen. Thromboembolische Komplikationen treten ferner auf bei mikroangiopathischen hämolytischen Anämien, extrakorporalen Blutkreisläufen, wie Hämodialyse, sowie Herzklappenprothesen.

Thromboembolische Erkrankungen sind die häufigste Ursache von Morbidität und Mortalität in den meisten industrialisierten Ländern. Die Inzidenz von venösen Thromboembolien (VTE) wird auf höher als 1 Fall in 1000 Personen geschätzt [R.H. White, "The epidemiology of venous thromboembolism" Circulation 2003, 107 (Suppl. 1), 14-18]. Ungefähr 1.3 bis 4.1 aus 1000 Personen bekommen einen ersten Schlaganfall [V.L. Feigin, C.M. Lawes, D.A. Bennett, C.S. Anderson, Lancet Neurol. 2003, 2, 43-53] und jährlich ungefähr 5 aus 1000 Personen einen Myocardinfarkt [J. Fang, M.H. Alderman, Am. J. Med. 2002, 113, 208-214].

Die aus dem Stand der Technik bekannten Antikoagulantien, d.h. Stoffe zur Hemmung oder Verhinderung der Blutgerinnung, weisen verschiedene, oftmals gravierende Nachteile auf. Eine effiziente Behandlungsmethode bzw. Prophylaxe von thromboembolischen Erkrankungen erweist sich in der Praxis deshalb als sehr schwierig und unbefriedigend.

Für die Therapie und Prophylaxe von thromboembolischen Erkrankungen findet zum einen Heparin Verwendung, das parenteral oder subkutan appliziert wird. Aufgrund günstigerer pharrnakokinetischer Eigenschaften wird zwar heutzutage zunehmend niedermolekulares Heparin bevorzugt; allerdings können auch hierdurch die im folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Da Heparin gleichzeitig mehrere Faktoren der Blutgerinnungskaskade hemmt, kommt es zu einer unselektiven Wirkung. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen [Pschyrembel, Klinisches Wörterbuch, 257. Auflage, 1994, Walter de Gruyter Verlag, Seite 610, Stichwort "Heparin"; Römpp Lexikon Chemie, Version 1.5, 1998, Georg Thieme Verlag Stuttgart, Stichwort "Heparin"].

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung aber nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig [J. Hirsh, J. Dalen, D.R. Anderson et al., "Oral anticoagulants: Mechanism of action, clinical effectiveness, and optimal therapeutic range" Chest 2001, 119, 8S-21S; J. Ansell, J. Hirsh, J. Dalen et al., "Managing oral anticoagulant therapy" Chest 2001, 119, 22S-38S; P.S. Wells, A.M. Holbrook, N.R. Crowther et al., "Interactions of warfarin with drugs and food" Ann. Intern. Med. 1994, 121, 676-683].

In jüngster Zeit ist ein neuer Therapieansatz für die Behandlung und Prophylaxe von thromboembolischen Erkrankungen beschrieben worden. Ziel dieses neuen Therapieansatzes ist die Inhibierung von Faktor Xa. Entsprechend der zentralen Rolle, die Faktor Xa in der Blutgerinnungkaskade spielt, stellt Faktor Xa eines der wichtigsten Targets für antikoagulatorische Wirkstoffe dar [J. Hauptmann, J. Stürzebecher, Thrombosis Research 1999, 93, 203; S.A.V. Raghavan, M. Dikshit, "Recent advances in the status and targets of antithrombotic agents" Drugs Fut. 2002, 27, 669-683; H.A. Wieland, V. Laux, D. Kozian, M. Lorenz, "Approaches in anticoagulation: Rationales for target positioning" Curr. Opin. Investig. Drugs 2003, 4, 264-271; U.J. Ries, W. Wienen, "Serine proteases as targets for antithrombotic therapy" Drugs Fut. 2003, 28, 355-370; L.-A. Linkins, J.I. Weitz, "New anticoagulant therapy" Annu. Rev. Med. 2005, 56, 63-77].

Dabei ist gezeigt worden, dass verschiedene, sowohl peptidische wie nicht-peptidische Verbindungen in Tiermodellen als Faktor Xa-Inhibitoren wirksam sind. Eine große Anzahl von direkten Faktor Xa-Inhibitoren ist bislang bekannt [J.M. Walenga, W.P. Jeske, D. Hoppensteadt, J. Fareed, "Factor Xa Inhibitors: Today and beyond" Curr. Opin. Investig. Drugs 2003, 4, 272-281; J. Ruef, H.A. Katus, "New antithrombotic drugs on the horizon" Expert Opin. Investig. Drugs 2003, 12, 781-797; M.L. Quan, J.M. Smallheer, "The race to an orally active Factor Xa inhibitor: Recent advances" Curr. Opin. Drug Discovery & Development 2004, 7, 460-469]. Faktor Xa-Inhibitoren mit Oxazolidinon-Partialstruktur sind in WO 01/47919, WO 02/064575 und WO 03/000256 beschrieben.

Die Aufgabe der vorliegenden Erfindung liegt in der Bereitstellung neuer Substanzen zur Bekämpfung von Erkrankungen, insbesondere von thromboembolischen Erkrankungen.

Gegenstand der vorliegenden Erfindung sind Verbindungen der allgemeinen Formel (I) 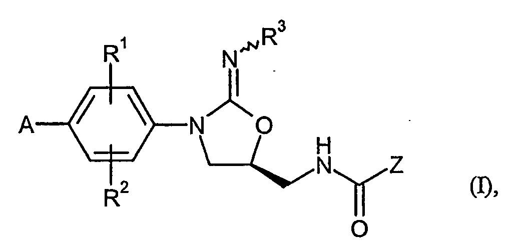 in welcher
- A: für eine Gruppe der Formel 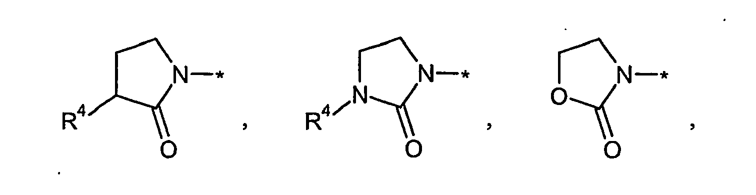 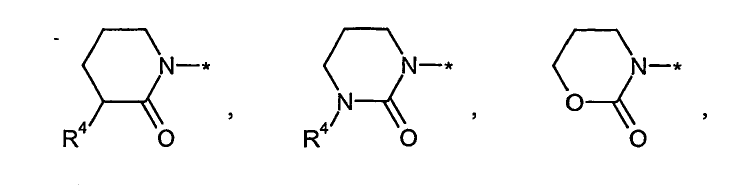 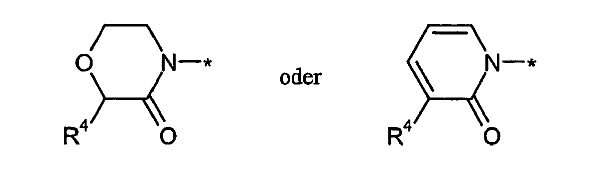 steht, worin
R⁴ Wasserstoff, (C₁-C₆)-Alkyl, (C₃-C₇)-Cycloalkyl, Hydroxy, (C₁-C₆)-Alkoxy, Amino, Mono- oder Di-(C₁-C₆)-alkylamino, (C₃-C₇)-Cycloalkylamino, (C₁-C₆)-Alkanoylamino oder (C₁-C₆)-Alkoxycarbonylamino bedeutet, wobei
(C₁-C₆)-Alkyl, (C₁-C₆)-Alkoxy, Mono- und Di-(C₁-C₆)-alkylamino ihrerseits jeweils durch Hydroxy, (C₁-C₄)-Alkoxy, Amino, Mono- oder Di-(C₁-C₄)-alkylamino, (C₃-C₇)-Cycloalkylamino oder einen 4- bis 7-gliedrigen gesättigten, über ein N-Atom gebundenen Heterocyclus, der ein Ringglied aus der Reihe N-R⁵ oder O enthalten kann, worin
R⁵ Wasserstoff oder (C₁-C₄)-Alkyl bedeutet, substituiert sein können, und * die Verknüpfungsstelle mit dem Phenylring bedeutet,
oder
- A: für eine Gruppe der Formel -C(=O)-NR⁶R⁷ steht, worin
R⁶ und R⁷ gleich oder verschieden sind und unabhängig voneinander (C₁-C₆)-Alkyl oder (C₃-C₇)-Cycloalkyl bedeuten
oder
R⁶ und R⁷ gemeinsam mit dem Stickstoffatom, an das sie gebunden sind, einen 4- bis 6-gliedrigen gesättigten oder partiell ungesättigten Heterocyclus bilden, der ein Ringglied aus der Reihe N-R⁸ oder O enthalten und durch (C₁-C₆)-Alkyl, Hydroxy, (C₁-C₆)-Alkoxy, Oxo, Amino, Mono- oder Di-(C₁-C₆)-alkylamino substituiert sein kann, worin
R⁸ Wasserstoff oder (C₁-C₆)-Alkyl bedeutet,
wobei alle genannten (C₁-C₆)-Alkylgruppen ihrerseits durch Hydroxy, (C₁-C₄)-Alkoxy, Amino, Mono- oder Di-(C₁-C₄)-alkylamino oder (C₃-C₇)-Cycloalkylamino substituiert sein können,
- Z: für Phenyl, Pyridyl, Pyrimidinyl, Pyrazinyl, Thienyl, Furyl oder Pyrrolyl steht, die jeweils ein- oder zweifach, gleich oder verschieden, durch Substituenten ausgewählt aus der Gruppe Halogen, Cyano, (C₁-C₄)-Alkyl, welches seinerseits durch Hydroxy oder Amino substituiert sein kann, Ethinyl, Cyclopropyl und Amino substituiert sein können,
- R¹ und R²: gleich oder verschieden sind und unabhängig voneinander für Wasserstoff, Halogen, Cyano, (C₁-C₄)-Alkyl, Cyclopropyl, Trifluormethyl, Hydroxy, (C₁-C₄)-Alkoxy, Trifluormethoxy, Amino, Mono- oder Di-(C₁-C₄)-alkylamino stehen, wobei
(C₁-C₄)-Alkyl und (C₁-C₄)-Alkoxy ihrerseits jeweils durch Hydroxy, (C₁-C₄)-Alkoxy, Amino, Mono- oder Di-(C₁-C₄)-alkylamino oder (C₃-C₇)-Cycloalkylamino substituiert sein können,
und
- R³: für Wasserstoff, (C₁-C₆)-Alkyl oder Cyano steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl und n-Hexyl.
(C₃-C₇)-Cycloalkyl steht im Rahmen der Erfindung für eine monocyclische Cycloalkylgruppe mit 3 bis 7 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.
(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy und tert.-Butoxy.
(C₁-C₆)-Alkanoyl [(C₁-C₆)-Acyl] steht im Rahmen der Erfindung für einen geradkettigen oder verzweigten Alkylrest mit 1 bis 6 Kohlenstoffatomen, der in der 1-Position ein doppelt gebundenes Sauerstoffatom trägt und über die 1-Position verknüpft ist. Bevorzugt ist ein geradkettiger oder verzweigter Alkanoylrest mit 1 bis 4 Kohlenstoffatomen. Beispielhaft und vorzugsweise seien genannt: Formyl, Acetyl, Propionyl, n-Butyryl, iso-Butyryl und Pivaloyl.
(C₁-C₆)-Alkoxycarbonyl steht im Rahmen der Erfmdung für einen geradkettigen oder verzweigten Alkoxyrest mit 1 bis 6 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Mono-(C₁-C₆)-Alkylamino und Mono-(C₁-C₄)-Alkylamino stehen im Rahmen der Erfindung für eine Amino-Gruppe mit einem geradkettigen oder verzweigten Alkylsubstituenten, der 1 bis 6 bzw. 1 bis 4 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino und tert.-Butylamino.
Di-(C₁-C₆)-Alkylamino und Di-(C₁-C₄)-Alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N,N*-Dimethylamino, *N,N*-Diethylamino, *N*-Ethyl-*N*-methylamino, *N*-Methyl-*N*-n-propylamino, *N*-Isopropyl-*N*-n-propylamino, *N*-tert.-Butyl-*N*-methylamino, *N-*Ethyl-*N*-n-pentylamino und *N*-n-Hexyl-*N-*methylamino.
(C₃-C₇)-Cycloalkylamino steht im Rahmen der Erfindung für eine Amino-Gruppe mit einem Cycloalkyl-Substituenten, der 3 bis 7 Kohlenstoffatome aufweist. Bevorzugt ist ein Cycloalkylamino-Rest mit 3 bis 6 Kohlenstoffatomen. Beispielhaft und vorzugsweise seien genannt: Cyclopropylamino, Cyclobutylamino, Cyclopentylamino, Cyclohexylamino und Cycloheptylamino.
(C₁-C₆)-Alkanoylamino steht im Rahmen der Erfindung für eine Amino-Gruppe mit einem geradkettigen oder verzweigten Alkanoyl-Substituenten, der 1 bis 6 Kohlenstoffatome aufweist und über die Carbonylgruppe verknüpft ist. Bevorzugt ist ein Alkanoylamino-Rest mit 1 bis 4 Kohlenstoffatomen. Beispielhaft und vorzugsweise seien genannt: Formamido, Acetamido, Propionamido, n-Butyramido und Pivaloylamido.
(C₁-C₆)-Alkoncarbonylamino steht im Rahmen der Erfindung für eine Amino-Gruppe mit einem geradkettigen oder verzweigten Alkoxycarbonyl-Substituenten, der im Alkoxyrest 1 bis 6 Kohlenstoffatome aufweist und über die Carbonylgruppe verknüpft ist. Bevorzugt ist ein Alkoxycarbonylamino-Rest mit 1 bis 4 Kohlenstoffatomen in der Alkoxy-Gruppe. Beispielhaft und vorzugsweise seien genannt: Methoxycarbonylamino, Ethoxycarbonylamino, n-Propoxycarbonylamino und tert.-Butoxycarbonylamino.
Ein 4- bis 7-gliedriger Heterocyclus steht im Rahmen der Erfindung für einen gesättigten Heterocyclus mit 4 bis 7 Ringatomen, der ein Ring-Stickstoffatom enthält, über dieses verknüpft ist und ein weiteres Heteroatom aus der Reihe N oder O als Ringglied enthalten kann. Bevorzugt ist ein 5-oder 6-gliedriger gesättigter, N-verknüpfter Heterocyclus, der ein weiteres Heteroatom aus der Reihe N oder O enthalten kann. Beispielhaft seien genannt: Azetidinyl, Pyrrolidinyl, Oxazolidinyl, Imidazolidinyl, Piperidinyl, Piperazinyl, Morpholinyl, Azepinyl und 1,4-Diazepinyl. Besonders bevorzugt sind Pyrrolidinyl, Piperidinyl, Piperazinyl und Morpholinyl,
Ein 4- bis 6-gliedriger gesättigter oder partiell ungesättigter Heterocyclus steht im Rahmen der Erfindung für einen Heterocyclus mit 4 bis 6 Ringatomen, der ein Ring-Stickstoffatom enthält, über dieses verknüpft ist, ein weiteres Heteroatom aus der Reihe N oder O als Ringglied enthalten kann und gesättigt ist oder eine Doppelbindung enthält. Bevorzugt ist ein 5- oder 6-gliedriger gesättigter oder partiell ungesättigter, N-verknüpfter Heterocyclus, der ein weiteres Heteroatom aus der Reihe N oder O enthalten kann. Beispielhaft seien genannt: Azetidinyl, Pyrrolidinyl, Oxazolidinyl, Imidazolidinyl, Pyrrolinyl, Pyrazolinyl, Imidazolinyl, 4-Oxazolinyl, Isoxazolinyl, Piperidinyl, Piperazinyl, Morpholinyl, Tetrahydropyridinyl und Tetrahydropyrimidinyl. Besonders bevorzugt sind Pyrrolidinyl, Pyrrolinyl, Piperidinyl, Piperazinyl und Morpholinyl.
Halogen schließt im Rahmen der Erfindung Fluor, Chlor, Brom und Iod ein. Bevorzugt sind Fluor oder Chlor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt sind Verbindungen der Formel (I), in welcher
- A: für eine Gruppe der Formel 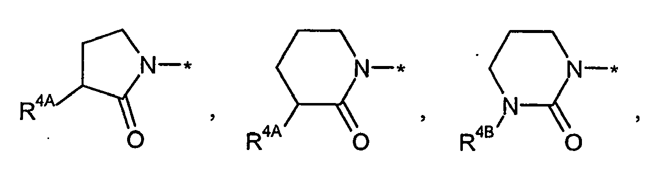 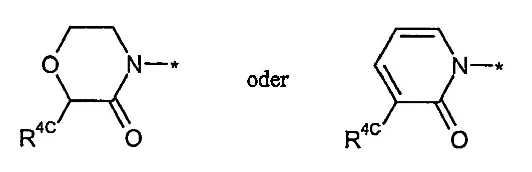 steht, worin
R^{4A} Wasserstoff, Hydroxy, Methoxy oder Amino,
R^{4B} Methyl oder Ethyl, welche jeweils durch Hydroxy, Amino, Pyrrolidino oder Cyclopropylamino substituiert sein können, oder Amino,
R^{4c} Wasserstoff, Methyl oder Ethyl, wobei Methyl und Ethyl jeweils durch Hydroxy, Amino, Pyrrolidino oder Cyclopropylamino substituiert sein können,
und
* die Verknüpfungsstelle mit dem Phenylring bedeuten,
- Z: für eine Gruppe der Formel 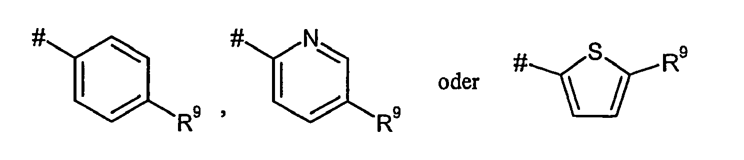 steht, worin
R⁹ Fluor, Chlor, Methyl oder Ethinyl
und
# die Verknüpfungsstelle mit der Carbonylgruppe bedeutet,
- R¹: für Wasserstoff,
- R²: für Wasserstoff, Fluor oder Methyl,
und
- R³: für Wasserstoff oder (C₁-C₄)-Alkyl stehen,
sowie ihre Salze, Solvate und Solvate der Salze.

Besonders bevorzugt sind Verbindungen der Formel (I), in welcher
- A: für eine Gruppe der Formel 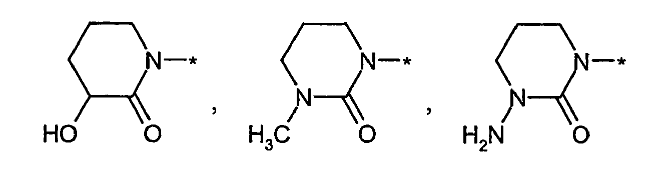 oder 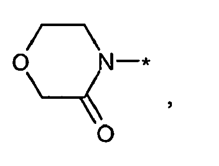 worin * die Verlrnüpfungsstelle mit dem Phenylring bedeutet,
- Z: für eine Gruppe der Formel 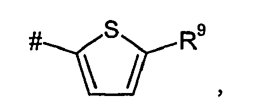 worin
R⁹ Fluor, Chlor oder Methyl
und
# die Verknüpfungsstelle mit der Carbonylgruppe bedeutet,
- R¹: für Wasserstoff,
- R²: für Wasserstoff, Fluor oder Methyl,
und
- R³: für Wasserstoff stehen,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), in welcher R³ für Wasserstoff steht, **dadurch gekennzeichnet, dass** man (2*S*)-3-Aminopropan-1,2-diol der Formel (II) 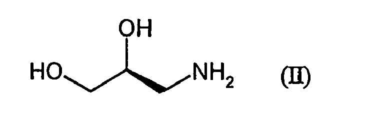 in einem inerten Lösungsmittel in Gegenwart einer Base mit einer Verbindung der Formel (III) 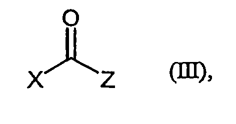 in welcher Z die oben angegebene Bedeutung aufweist und
- X: für eine geeignete Abgangsgruppe wie beispielsweise Halogen, bevorzugt für Chlor steht,
zu Verbindungen der Formel (IV) 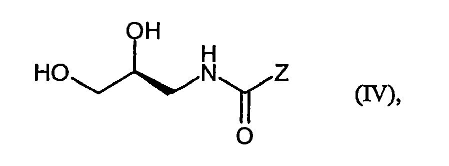 in welcher Z die oben angegebene Bedeutung aufweist,
umsetzt, anschließend mit Hilfe von Bromwasserstoffsäure in Essigsäure, gegebenenfalls unter Zusatz von Essigsäureanhydrid, in Verbindungen der Formel (V) 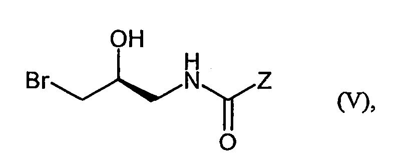 in welcher Z die oben angegebene Bedeutung aufweist,
überführt, diese dann in einem inerten Lösungsmittel in Gegenwart einer Base zu Verbindungen der Formel (VI) 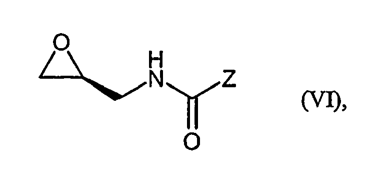 in welcher Z die oben angegebene Bedeutung aufweist,
cyclisiert, nachfolgend in einem inerten Lösungsmittel, gegebenenfalls in Gegenwart einer Protonen- oder Lewis-Säure, mit einer Verbindung der Formel (VII) 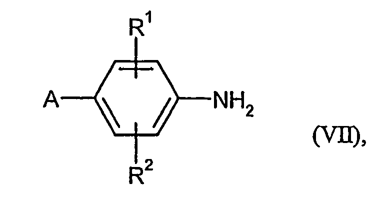 in welcher A, R¹ und R² die oben angegebenen Bedeutungen aufweisen,
zu Verbindungen der Formel (VIII) 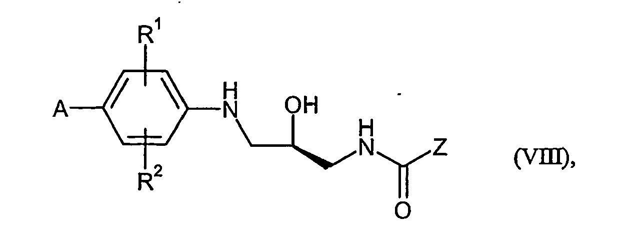 in welcher A, Z, R¹ und R² die oben angegebenen Bedeutungen aufweisen,
umsetzt und diese dann in einem inerten Lösungsmittel mit Bromcyan, gegebenenfalls in Gegenwart einer Säure, zu Verbindungen der Formel (I-A) 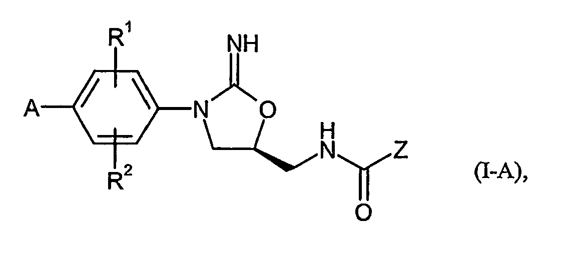 in welcher A, Z, R¹ und R² die oben angegebenen Bedeutungen aufweisen,
reagiert
und die Verbindungen der Formel (I-A) gegebenenfalls mit den entsprechenden (i) Lösungsmitteln und/oder (ii) Basen oder Säuren zu ihren Solvaten, Salzen und/oder Solvaten der Salze umsetzt.

Die erfindungsgemäßen Verbindungen der Formel (I), in welcher R³ nicht für Wasserstoff steht, können ausgehend von den Verbindungen der Formel (VIII) in Analogie zu literaturbekannten Verfahren hergestellt werden [vgl. z.B. für R³ = Cyano: a) R. Evers, M. Michalik, J. Prakt. Chem. 1991, 333, 699-710; N. Maezaki, A. Furusawa, S. Uchida, T. Tanaka, Tetrahedron 2001, 57, 9309-9316; G. Berecz, J. Reiter, G. Argay, A. Kalman, J. Heterocycl. Chem. 2002, 39, 319-326; b) R. Mohr, A. Buschauer, W. Schunack, Arch. Pharm. (Weinheim Ger.) 1988, 321, 221-227; für R³ = Alkyl: a) V.A. Vaillancourt et al., J. Med. Chem. 2001, 44, 1231-1248; b) F.B. Dains et al., J. Amer. Chem. Soc. 1925, 47, 1981-1989; J. Amer. Chem. Soc. 1922, 44, 2637-2643 und T. Shibanuma, M. Shiono, T. Mukaiyama, Chem. Lett. 1977, 575-576; siehe auch Syntheseschemata 2 und 3].

Die erfindungsgemäßen Verbindungen können gegebenenfalls auch durch weitere Umwandlungen von funktionellen Gruppen einzelner Substituenten, insbesondere den unter R¹, R², R⁴, R⁶ und R⁷ aufgeführten, ausgehend von den nach obigem Verfahren erhaltenen Verbindungen der Formel (I) hergestellt werden. Diese Umwandlungen werden nach üblichen Methoden durchgeführt und umfassen beispielsweise Reaktionen wie Alkylierung, Aminierung, Acylierung, Veresterung, Esterspaltung, Amid-Bildung, Oxidation oder Reduktion sowie die Einführung und Entfernung temporärer Schutzgruppen.

Inerte Lösungsmittel für den Verfahrensschritt (II) + (III) → (IV) sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, 2-Methyltetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Aceton, Dimethylformamid, Dimethylsulfoxid, *N,N*'-Dimethylpropylenharnstoff (DMPU), *N*-Methylpyrrolidon (NMP), Acetonitril, Pyridin oder auch Wasser. Ebenso ist es möglich, Gemische der genannten Lösungsmittel einzusetzen. Bevorzugt wird ein Gemisch aus Wasser, 2-Methyltetrahydrofuran und Toluol verwendet.

### (2S)-3-Aminopropan-1,2-diol (II) kann bei diesem Verfahrensschritt als freie Base oder als Säureadditionssalz eingesetzt werden; bevorzugt wird das Hydrochlorid verwendet.

Als Base für den Verfahrensschritt (II) + (III) → (IV) eignen sich übliche anorganische oder organische Basen. Hierzu gehören insbesondere Alkalihydrogencarbonate wie Natrium- oder Kaliumhydrogencarbonat, Alkali- oder Erdalkalicarbonate wie Lithium-, Natrium-, Kalium-, Calcium- oder Cäsiumcarbonat, oder organische Amine wie Triethylamin, *N*-Methylmorpholin, *N-*Methylpiperidin, *N,N-*Diisopropylethylamin oder Pyridin. Bevorzugt wird Natriumhydrogencarbonat verwendet.

Die Base wird hierbei in einer Menge von 1 bis 3 Mol, bevorzugt in einer Menge von 1 bis 2 Mol, bezogen auf 1 Mol der Verbindung der Formel (II) bzw. dessen Hydrochlorid, eingesetzt. Die Reaktion erfolgt im Allgemeinen in einem Temperaturbereich von 0°C bis +50°C, bevorzugt von +5°C bis +30°C.

Die Umsetzung (IV) → (V) wird mit 1 bis 5, vorzugsweise 3 bis 5 Äquivalenten wässriger Bromwasserstoffsäure in Essigsäure, gegebenenfalls unter Zusatz von Essigsäureanhydrid, durchgeführt. Die Reaktion erfolgt im Allgemeinen in einem Temperaturbereich von +20°C bis +100°C, bevorzugt von +50°C bis +80°C.

Inerte Lösungsmittel für den Verfahrensschritt (V) → (VI) sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, 2-Methyltetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid, Dimethylsulfoxid, *N,N*'-Dimethylpropylenharnstoff (DMPU), *N-*Methylpyrrolidon (NMP) oder Acetonitril. Ebenso ist es möglich, Gemische der genannten Lösungsmittel einzusetzen. Bevorzugt werden Dichlormethan oder Tetrahydrofuran verwendet.

Als Base für die Cyclisierung (V) → (VI) eignen sich übliche anorganische Basen. Hierzu gehören insbesondere Alkalihydrogencarbonate wie Natrium- oder Kaliumhydrogencarbonat, oder Alkali-oder Erdalkalicarbonate wie Lithium-, Natrium-, Kalium-, Calcium- oder Cäsiumcarbonat. Bevorzugt wird Kaliumcarbonat verwendet.

Die Base wird hierbei in einer Menge von 1 bis 5 Mol, bevorzugt in einer Menge von 3 bis 5 Mol, bezogen auf 1 Mol der Verbindung der Formel (V), eingesetzt. Die Reaktion erfolgt im Allgemeinen in einem Temperaturbereich von 0°C bis +50°C, bevorzugt von +10°C bis +30°C.

Inerte Lösungsmittel für den Verfahrensschritt (VI) + (VII) → (VIII) sind beispielsweise Alkohole wie Methanol, Ethanol, n-Propanol, iso-Propanol, n-Butanol oder tert.-Butanol, Ether wie Diethylether, Dioxan, Tetrahydrofuran, 2-Methyltetrahydrofuran, Glykoldimethylether oder Diethylenglykoldimethylether, oder andere Lösungsmittel wie Aceton, Dimethylformamid, Dimethylsulfoxid, *N,N*'-Dimethylpropylenharnstoff (DMPU), *N*-Methylpyrrolidon (NMP), Acetonitril oder auch Wasser. Ebenso ist es möglich, Gemische der genannten Lösungsmittel einzusetzen. Bevorzugt werden Dioxan, Tetrahydrofuran, Ethanol oder deren Gemische mit Wasser verwendet.

Der Verfahrensschritt (VI) + (VII) → (VIII) kann wahlweise auch unter Zusatz einer katalytischen Menge einer Protonen-Säure, wie beispielsweise p-Toluolsulfonsäure, oder einer Lewis-Säure, wie beispielsweise Ytterbium(III)trifluormethansulfonat, durchgeführt werden.

Die Umsetzung (VI) + (VII) → (VIII) erfolgt im Allgemeinen in einem Temperaturbereich von 0°C bis +100°C, bevorzugt von +20°C bis +80°C.

Inerte Lösungsmittel für den Verfahrensschritt (VIII) → (I-A) sind beispielsweise Halogenkohlenwasserstoffe wie Dichlormethan, Trichlormethan, Tetrachlormethan, Trichlorethan, Tetrachlorethan oder 1,2-Dichlorethan, Ether wie Diethylether, Dioxan oder Tetrahydrofuran, Kohlenwasserstoffe wie Benzol, Xylol, Toluol, Hexan, Cyclohexan oder Erdölfraktionen, oder andere Lösungsmittel wie Acetonitril. Ebenso ist es möglich, Gemische der genannten Lösungsmittel einzusetzen. Bevorzugt wird Tetrahydrofuran oder Acetonitril verwendet.

Der Verfahrensschritt (VIII) → (I-A) kann vorteilhaft unter Zusatz einer starken anorganischen oder organischen Säure durchgeführt werden. Hierzu gehören insbesondere Säuren wie Fluorwasserstoff, Chlorwasserstoff, Bromwasserstoff, Methansulfonsäure, Trifluormethansulfonsäure oder Trifluoressigsäure.

Die Umsetzung (VIII) → (I-A) erfolgt im Allgemeinen in einem Temperaturbereich von -20°C bis +50°C, bevorzugt von 0°C bis +40°C.

Alle Verfahrensschritte können bei normalem, erhöhtem oder bei erniedrigtem Druck durchgeführt werden (z.B. von 0.5 bis 5 bar). Im Allgemeinen arbeitet man bei Normaldruck.

Die Verbindungen der Formeln (II), (III) und (VII) sind kommerziell erhältlich, literaturbekannt oder können in Analogie zu literaturbekannten Verfahren hergestellt werden.

Die Herstellung der erfindungsgemäßen Verbindungen kann durch die folgenden Syntheseschemata veranschaulicht werden:

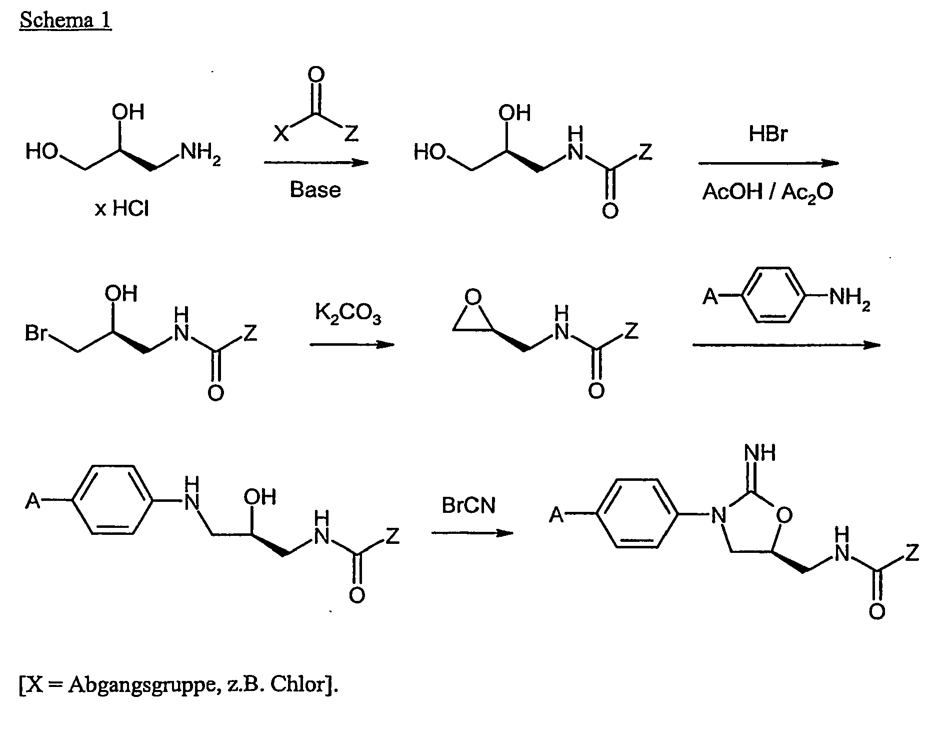

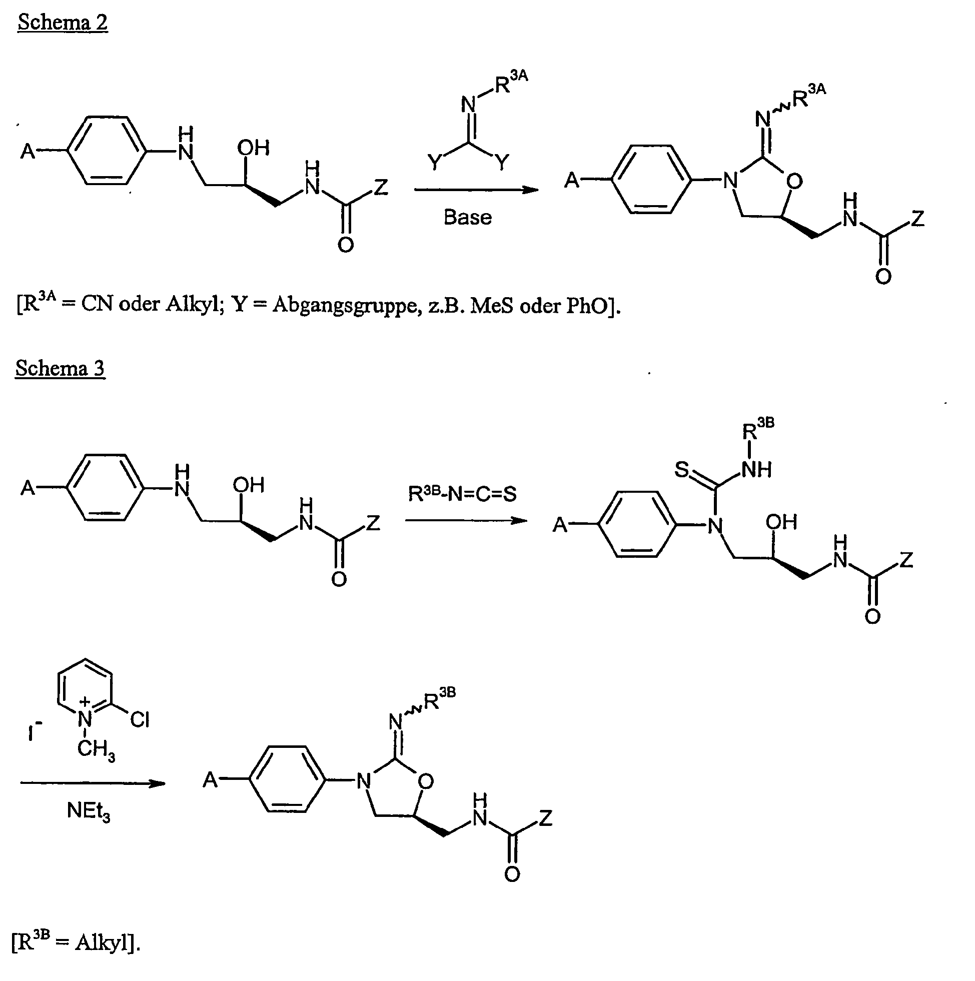

Die erfindungsgemäßen Verbindungen zeigen ein nicht vorhersehbares, wertvolles pharmakologisches Wirkspektrum, insbesondere eine hohe Wirkstärke.

Sie eignen sich daher zur Verwendung als Arzneimittel zur Behandlung und/oder Prophylaxe von Krankheiten bei Menschen und Tieren.

Bei den erfindungsgemäßen Verbindungen handelt es sich um selektive Inhibitoren des Blutgerinnungsfaktors Xa, die insbesondere als Antikoagulantien wirken.

Darüber hinaus verfügen die erfindungsgemäßen Verbindungen über günstige physikochemische Eigenschaften, wie beispielsweise eine gute Löslichkeit in Wasser und physiologischen Medien, was für ihre therapeutische Anwendung von Vorteil ist.

Weiterer Gegenstand der vorliegenden Erfindung ist der Einsatz der erfindungsgemäßen Verbindungen zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina pectoris, instabile Angina pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die Substanzen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen. Darüber hinaus sind die erfindungsgemäßen Verbindungen zur Behandlung der disseminierten intravasalen Gerinnung (DIC) geeignet.

Thromboembolische Komplikationen treten ferner auf bei mikroangiopathischen hämolytischen Anämien, extrakorporalen Blutkreisläufen, wie Hämodialyse, sowie Herzklappenprothesen.

Außerdem kommen die erfindungsgemäßen Verbindungen auch für die Prophylaxe und/oder Behandlung von atherosklerotischen Gefäßerkrankungen und entzündlichen Erkrankungen wie rheumatische Erkrankungen des Bewegungsapparats in Betracht, darüber hinaus ebenso für die Prophylaxe und/oder Behandlung der Alzheimer'schen Erkrankung. Außerdem können die erfindungsgemäßen Verbindungen zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Die erfindungsgemäßen Verbindungen können darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor Xa enthalten.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer antikoagulatorisch wirksamen Menge der erfindungsgemäßen Verbindung.

Weiterer Gegenstand der vorliegenden Erfindung ist ein Verfahren zur Verhinderung der Blutkoagulation *in vitro,* insbesondere bei Blutkonserven oder biologischen Proben, die Faktor Xa enthalten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren;
- Koronartherapeutika/Vasodilatatoren, insbesondere ACE-(Angiotensin-Converting-Enzyme)-Inhibitoren; AII-(Angiotensin II)-Rezeptor-Antagonisten; β-Adrenozeptor-Antagonisten; alpha-1-Adrenozeptor-Antagonisten; Diuretika; Calciumkanalblocker; Substanzen, die eine Erhöhung von cyclischem Guanosinmonophosphat (cGMP) bewirken, wie beispielsweise Stimulatoren der löslichen Guanylatcyclase;
- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI);
- antikoagulatorisch wirksame Substanzen (Antikoagulantien);
- plättchenaggregationshemmende Substanzen (Plättchenaggregationshemmer, Thrombozytenaggregationshemmer);
- Fibrinogen-Rezeptor-Antagonisten (Glycoprotein-IIb/IIIa-Antagonisten).

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfmdungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- Ac: Acetyl
- DMSO: Dimethylsulfoxid
- d. Th.: der Theorie (bei Ausbeute)
- ESI: Elektrospray-Ionisation (bei MS)
- Et: Ethyl
- h: Stunde(n)
- HPLC: Hochdruck-, Hochleistungsflüssigchromatographie
- LC-MS: Flüssigchromatographie-gekoppelte Massenspektroskopie
- Me: Methyl
- min: Minute(n)
- MS: Massenspektroskopie
- NMR: Kernresonanzspektroskopie
- Ph: Phenyl
- RP: reverse phase (bei HPLC)
- Rₜ: Retentionszeit (bei HPLC)
- THF: Tetrahydrofuran

### LC-MS- und HPLC-Methoden:

### Methode 1:

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2:

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 9 min 0% B → 9.2 min 2% B → 10 min 2% B; Fluss: 0.75 ml/min; Säulentemperatur: 30°C; UV-Detektion: 210 nm.

### Ausgangsverbindungen:

### Beispiel 1A

5-Chlorthiophen-2-carbonsäurechlorid

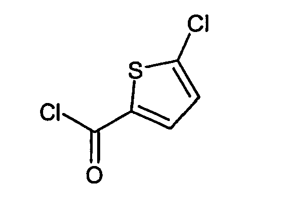

Die Darstellung der Titelverbindung erfolgt durch Umsetzung von 5-Chlorthiophen-2-carbonsäure mit Thionylchlorid, siehe R. Aitken et al., Arch. Pharm. (Weinheim Ger.) 1998, 331, 405-411.

### Beispiel 2A

4-(4-Aminophenyl)morpholin-3-on

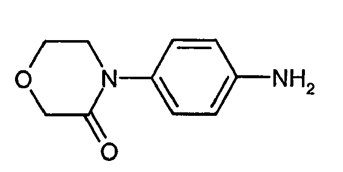

Die Darstellung der Titelverbindung erfolgt durch Umsetzung von 4-Fluornitrobenzol mit Morpholin-3-on [J.-M. Lehn, F. Montavon, Helv. Chim. Acta 1976, 59, 1566-1583] und anschließende Reduktion des erhaltenen 4-(4-Nitrophenyl)morpholin-3-ons (siehe WO 01/47919, Ausgangsverbindungen I bzw. II, S. 55-57).

### Beispiel 3A

5-Chlor-*N*-[(2*S*)-2-oxiranylmethyl]-2-thiophencarboxamid

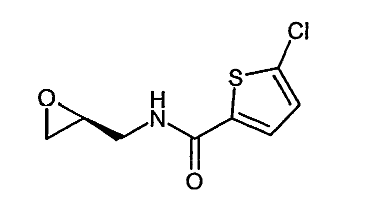

Die Darstellung der Titelverbindung erfolgt wie in WO 2004/101557 (Beispiel 6A) beschrieben durch (i) Acylierung von (2S)-3-Aminopropan-1,2-diol-Hydrochiorid mit 5-Chlorthiophen-2-carbonsäurechlorid in Gegenwart von Natriumhydrogencarbonat als Base, (ii) Hydroxy-Brom-Austausch mit Hilfe von Bromwasserstoffsäure in Essigsäure/Essigsäureanhydrid und (iii) Epoxidbildung in Gegenwart von Kaliumcarbonat als Base.

### Ausführungsbeispiele:

### Beispiel 1

5-Chlor-*N*-({(5*S*)-2-imino-3-[4-(3-oxomorpholin-4-yl)phenyl]-1,3-oxazolidin-5-yl}methyl)-thiophen-2-carboxamid

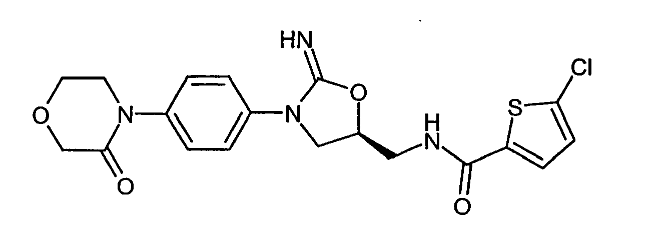

### Stufe a): 5-Chlor-N-((2R)-2-hydroxy-3-{[4-(3-oxo-4-morpholinyl)phenyl]amino}propyl)-2-thiophencarboxamid

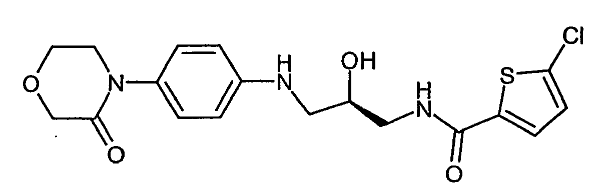

6.18 g (32 mmol) 4-(4-Aminophenyl)morpholin-3-on (Beispiel 2A) und 7.00 g (32 mmol) 5-Chlor-*N*-[(2*S*)-2-oxiranylmethyl]-2-thiophencarboxamid (Beispiel 3A) werden in 130 ml Ethanol/Wasser (9:1) suspendiert und bei 75°C über Nacht gerührt (Bildung einer Lösung). Die Lösung wird im Eisbad abgekühlt, der ausgefallene weiße Niederschlag abfiltriert, mit Diethylether gewaschen und im Hochvakuum getrocknet. Man erhält 4.98 g der Titelverbindung. Einengen der Mutterlauge, nochmalige Zugabe von 3.5 g (16 mmol) 5-Chlor-*N*-[(2*S*)-2-oxiranylmethyl]-2-thiophencarboxamid in 50 ml Ethanol/Wasser (9:1), erneutes Rühren bei 75°C über Nacht und Filtration des nach Abkühlen im Eisbad ausgefallenen Niederschlags ergibt weitere 3.44 g der Titelverbindung.
Ausbeute: 8.42 g insgesamt (62% d. Th.)
LC-MS (Methode 1): Rₜ = 1.46 min;
MS (ESIpos): m/z = 410 [M+H]⁺;
¹H-NMR (300 MHz, DMSO-d₆): δ = 8.60 (t, 1H), 7.69 (d, 1H), 7.18 (d, 1H), 7.02 (d, 2H), 6.59 (d, 2H), 5.65 (t, 1H), 5.08 (d, 1H), 4.13 (s, 2H), 3.91 (dd, 2H), 3.87-3.74 (m, 1H), 3.59 (m, 2H), 3.30-2.90 (m, 4H).

### Stufe b): 5-Chlor-N-({(5S)-2-imino-3-[4-(3-oxomorpholin-4-yl)phenyl]-1,3-oxazolidin-5-yl}-methyl)thiophen-2-carboxamid

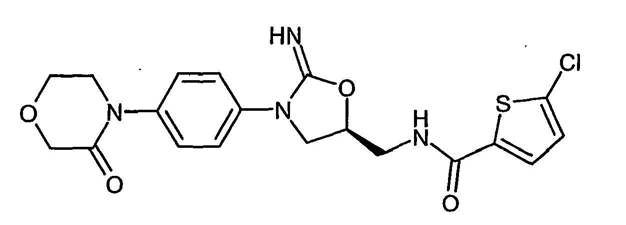

700 mg (1.71 mmol) 5-Chlor-*N*-((2*R*)-2-hydroxy-3-{[4-(3-oxo-4-morpholinyl)phenyl]amino}propyl)-2-thiophencarboxamid aus Stufe a) werden unter Argon bei Raumtemperatur 6 h lang in 13.7 ml einer 5-molaren Lösung von Bromcyan (68.3 mmol) in THF gerührt. Das Reaktionsgemisch wird eingeengt und der Rückstand (690 mg) mittels präparativer HPLC gereinigt [Säule: YMC Gel ODS-AQ S-11 µm; Laufmittel: Acetonitril/0.2%-ige Trifluoressigsäure 35:65]. Man erhält 491 mg (89% d. Th.) der Titelverbindung als Trifluoracetat-Salz. Die freie Base wird durch Rühren mit gesättigter wässriger Natriumhydrogencarbonat-Lösung in THF freigesetzt, und die Lösung wird anschließend mit Dichlormethan extrahiert. Die vereinigten organischen Phasen werden über Natriumsulfat getrocknet, filtriert und im Vakuum eingeengt. Man erhält 282 mg (38% d. Th.) der Titelverbindung als freie Base.
HPLC (Methode 2): Rₜ = 3.58 min;
MS (ESIpos): m/z = 435 [M+H]⁺;
¹H-NMR (400 MHz, DMSO-d₆): δ = 8.91 (t, 1H), 7.79 (d, 2H), 7.67 (d, 1H), 7.33 (d, 2H), 7.19 (d, 1H), 6.15 (s, 1H), 4.78-4.69 (m, 1H), 4.19 (s, 2H), 4.16-4.08 (m, 1H), 3.97 (dd, 2H), 3.79 (m, 1H), 3.69 (dd, 2H), 3.58-3.50 (m, 2H).

### B. Bewertung der pharmakologischen Wirksamkeit

Die erfindungsgemäßen Verbindungen wirken insbesondere als selektive Inhibitoren des Blutgerinnungsfaktors Xa und hemmen nicht oder erst bei deutlich höheren Konzentrationen auch andere Serinproteasen wie Plasmin oder Trypsin.

Als "selektiv" werden solche Inhibitoren des Blutgerinnungsfaktors Xa bezeichnet, bei denen die IC₅₀-Werte für die Faktor Xa-Inhibierung gegenüber den IC₅₀-Werten für die Inhibierung anderer Serinproteasen, insbesondere Plasmin und Trypsin, um mindestens das 100-fache kleiner sind, wobei bezüglich der Testmethoden für die Selektivität Bezug genommen wird auf die im folgenden beschriebenen Testmethoden der Beispiele B.a.1) und B.a.2).

Die vorteilhaften pharmakologischen Eigenschaften der erfindungsgemäßen Verbindungen können durch folgende Methoden festgestellt werden:

### a) Testbeschreibungen (in vitro)

### a.1) Messung der Faktor Xa-Hemmung:

Die enzymatische Aktivität von humanem Faktor Xa (FXa) wird über die Umsetzung eines für den FXa spezifischen chromogenen Substrats gemessen. Dabei spaltet der Faktor Xa aus dem chromogenen Substrat p-Nitroanilin ab. Die Bestimmungen werden wie folgt in Mikrotiterplatten durchgeführt:

Die Prüfsubstanzen werden in unterschiedlichen Konzentrationen in DMSO gelöst und für 10 Minuten mit humanem FXa (0.5 nmol/l gelöst in 50 mmol/l Tris-Puffer [*C*,*C*,*C*-Tris(hydroxymethyl)aminomethan], 150 mmol/l NaCl, 0.1% BSA [bovine serum albumine], pH = 8.3) bei 25°C inkubiert. Als Kontrolle dient reines DMSO. Anschließend wird das chromogene Substrat (150 µmol/l Pefachrome^{®} FXa der Firma Pentapharm) hinzugefügt. Nach 20 Minuten Inkubationsdauer bei 25°C wird die Extinktion bei 405 nm bestimmt. Die Extinktionen der Testansätze mit Prüfsubstanz werden mit den Kontrollansätzen ohne Prüfsubstanz verglichen und daraus die IC₅₀-Werte berechnet.

Das Ausführungsbeispiel 1 zeigt in diesem Test einen IC₅₀-Wert von 5.4 nM.

### a.2) Bestimmung der Selektivität:

Zum Nachweis der selektiven FXa-Inhibition werden die Prüfsubstanzen auf ihre Hemmung anderer humaner Serinproteasen wie Trypsin und Plasmin hin untersucht. Zur Bestimmung der enzymatischen Aktivität von Trypsin (500 mU/ml) und Plasmin (3.2 nmol/l) werden diese Enzyme in Tris-Puffer (100 mmol/l, 20 mmol/l CaCl₂, pH = 8.0) gelöst und für 10 Minuten mit Prüfsubstanz oder Lösungsmittel inkubiert. Anschließend wird durch Zugabe der entsprechenden spezifischen chromogenen Substrate (Chromozym Trypsin^{®} und Chromozym Plasmin^{®}; Fa. Roche Diagnostics) die enzymatische Reaktion gestartet und die Extinktion nach 20 Minuten bei 405 nm bestimmt. Alle Bestimmungen werden bei 37°C durchgeführt. Die Extinktionen der Testansätze mit Prüfsubstanz werden mit den Kontrollproben ohne Prüfsubstanz verglichen und daraus die IC₅₀-Werte berechnet.

Das Ausführungsbeispiel 1 zeigt in diesen Tests jeweils einen IC₅₀-Wert von >10 µM.

### a.3) Bestimmung der antikoagulatorischen Wirkung:

Die antikoagulatorische Wirkung der Prüfsubstanzen wird *in vitro* in Human- und Kaninchenplasma bestimmt. Dazu wird Blut unter Verwendung einer 0.11 molaren Natriumcitrat-Lösung als Vorlage in einem Mischungsverhältnis Natriumcitrat/Blut 1:9 abgenommen. Das Blut wird unmittelbar nach der Abnahme gut gemischt und 10 Minuten bei ca. 2500 g zentrifugiert. Der Überstand wird abpipettiert. Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Hemoliance^{®} RecombiPlastin, Fa. Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

### b) Bestimmung der antithrombotischen Wirkung (in vivo)

### b.1) Arteriovenöses Shunt-Modell (Kaninchen):

Nüchterne Kaninchen (Stamm: Esd: NZW) werden durch intramuskuläre Gabe einer Rompun/Ketavet-Lösung narkotisiert (5 mg/kg bzw. 40 mg/kg). Die Thrombusbildung wird in einem arteriovenösen Shunt in Anlehnung an die von C.N. Berry et al. [Semin. Thromb. Hemost. 1996, 22, 233-241] beschriebene Methode ausgelöst. Dazu werden die linke Vena jugularis und die rechte Arteria carotis freipräpariert. Ein extracorporaler Shunt wird mittels eines 10 cm langen Venenkatheders zwischen den beiden Gefäßen gelegt. Dieser Katheder ist in der Mitte in einen weiteren, 4 cm langen Polyethylenschlauch (PE 160, Becton Dickenson), der zur Erzeugung einer thrombogenen Oberfläche einen aufgerauhten und zu einer Schlinge gelegten Nylonfaden enthält, eingebunden. Der extrakorporale Kreislauf wird 15 Minuten lang aufrechterhalten. Dann wird der Shunt entfernt und der Nylonfaden mit dem Thrombus sofort gewogen. Das Leergewicht des Nylonfadens ist vor Versuchsbeginn ermittelt worden. Die Prüfsubstanzen werden vor Anlegung des extrakorporalen Kreislaufs entweder intravenös über eine Ohrvene oder oral mittels Schlundsonde verabreicht.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfmdungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 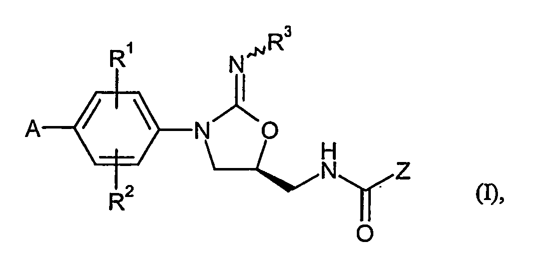 in welcher
A für eine Gruppe der Formel 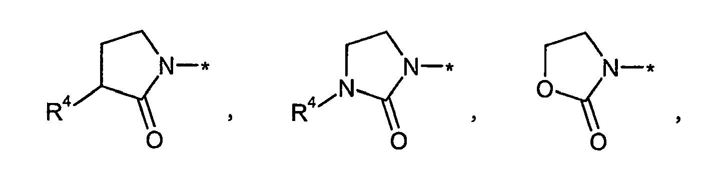 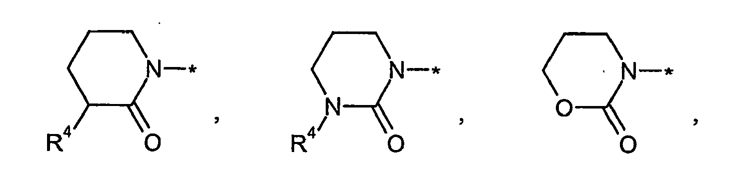 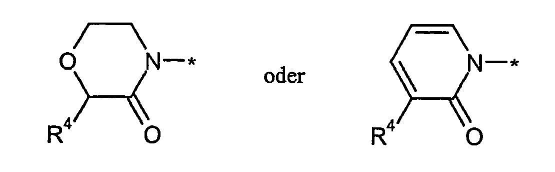 steht, worin
R⁴ Wasserstoff, (C₁-C₆)-Alkyl, (C₃-C₇)-Cycloalkyl, Hydroxy, (C₁-C₆)-Alkoxy, Amino, Mono- oder Di-(C₁-C₆)-alkylamino, (C₃-C₇)-Cyloalkylamino, (C₁-C₆)-Alkänoylamino oder (C₁-C₆)-Alkoxycarbonylamino bedeutet, wobei
(C₁-C₆)-Alkyl, (C₁-C₆)-Alkoxy, Mono- und Di-(C₁-C₆)-alkylamino ihrerseits jeweils durch Hydroxy, (C₁-C₄)-Alkoxy, Amino, Mono- oder Di-(C₁-C₄)-alkylamino, (C₃-C₇)-Cycloalkylamino oder einen 4- bis 7-gliedrigen gesättigten, über ein N-Atom gebundenen Heterocyclus, der ein Ringglied aus der Reihe N-R⁵ oder O enthalten kann, worin
R⁵ Wasserstoff oder (C₁-C₄)-Alkyl bedeutet,
substituiert sein können,
und * die Verknüpfungsstelle mit dem Phenylring bedeutet,
oder
A für eine Gruppe der Formel -C(=O)-NR⁶R⁷ steht, worin
R⁶ und R⁷ gleich oder verschieden sind und unabhängig voneinander (C₁-C₆)-Alkyl oder (C₃-C₇)-Cycloalkyl bedeuten
oder
R⁶ und R⁷ gemeinsam mit dem Stickstoffatom, an das sie gebunden sind, einen 4-bis 6-gliedrigen gesättigten oder partiell ungesättigten Heterocyclus bilden, der ein Ringglied aus der Reihe N-R⁸ oder O enthalten und durch (C₁-C₆)-Alkyl, Hydroxy, (C₁-C₆)-Alkoxy, Oxo, Amino, Mono- oder Di-(C₁-C₆)-alkylamino substituiert sein kann, worin
R⁸ Wasserstoff oder (C₁-C₆)-Alkyl bedeutet,
wobei alle genannten (C₁-C₆)-Alkylgruppen ihrerseits durch Hydroxy, (C₁-C₄)-Alkoxy, Amino, Mono- oder Di-(C₁-C₄)-alkylamino oder (C₃-C₇)-Cycloalkylamino substituiert sein können,
Z für Phenyl, Pyridyl, Pyrimidinyl, Pyrazinyl, Thienyl, Furyl oder Pyrrolyl steht, die jeweils ein- oder zweifach, gleich oder verschieden, durch Substituenten ausgewählt aus der Gruppe Halogen, Cyano, (C₁-C₄)-Alkyl, welches seinerseits durch Hydroxy oder Amino substituiert sein kann, Ethinyl, Cyclopropyl und Amino substituiert sein können,
R¹ und R² gleich oder verschieden sind und unabhängig voneinander für Wasserstoff, Halogen, Cyano, (C₁-C₄)-Alkyl, Cyclopropyl, Trifluormethyl, Hydroxy, (C₁-C₄)-Alkoxy, Trifluormethoxy, Amino, Mono- oder Di-(C₁-C₄)-alkylamino stehen, wobei
(C₁-C₄)-Alkyl und (C₁-C₄)-Alkoxy ihrerseits jeweils durch Hydroxy, (C₁-C₄)-Alkoxy, Amino, Mono- oder Di-(C₁-C₄)-alkylamino oder (C₃-C₇)-Cycloalkylamino substituiert sein können,
und
R³ für Wasserstoff, (C₁-C₆)-Alkyl oder Cyano steht,
sowie ihre Salze, Solvate und Solvate der Salze.

2. Verbindung der Formel (I) nach Anspruch 1, in welcher
A für eine Gruppe der Formel 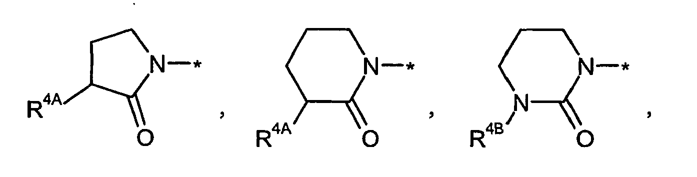 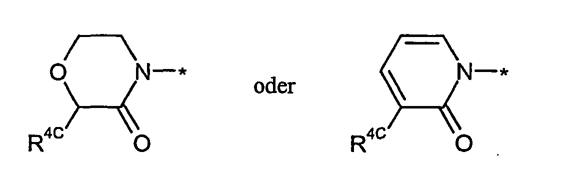 steht, worin
R^{4A} Wasserstoff, Hydroxy, Methoxy oder Amino,
R^{4B} Methyl oder Ethyl, welche jeweils durch Hydroxy, Amino, Pyrrolidino oder Cyclopropylamino substituiert sein können, oder Amino,
R^{4C} Wasserstoff, Methyl oder Ethyl, wobei Methyl und Ethyl jeweils durch Hydroxy, Amino, Pyrrolidino oder Cyclopropylamino substituiert sein können,
und
* die Verknüpfungsstelle mit dem Phenylring bedeuten,
Z für eine Gruppe der Formel 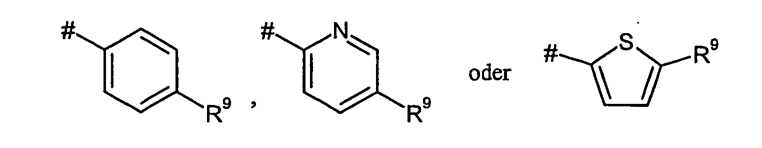 steht, worin
R⁹ Fluor, Chlor, Methyl oder Ethinyl und
# die Verknüpfungsstelle mit der Carbonylgruppe bedeutet,
R¹ für Wasserstoff,
R² für Wasserstoff, Fluor oder Methyl,
und
R³ für Wasserstoff oder (C₁-C₄)-Alkyl stehen,
sowie ihre Salze, Solvate und Solvate der Salze.

3. Verbindung der Formel (I) nach Anspruch 1 oder 2, in welcher
A für eine Gruppe der Formel 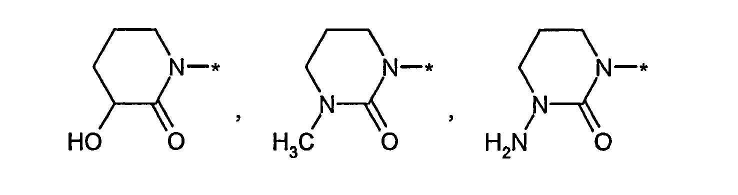 oder 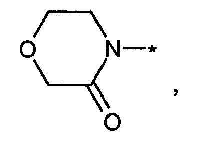 worin * die Verlrnüpfungsstelle mit dem Phenylring bedeutet,
Z für eine Gruppe der Formel 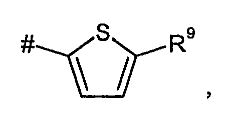 worin
R⁹ Fluor, Chlor oder Methyl
und
# die Verknüpfungsstelle mit der Carbonylgruppe bedeutet,
R¹ für Wasserstoff,
R² für Wasserstoff, Fluor oder Methyl,
und
R³ für Wasserstoff stehen,
sowie ihre Salze, Solvate und Solvate der Salze.

4. Verfahren zur Herstellung von Verbindungen der Formel (I), wie in Anspruch 1 definiert, in welcher R³ für Wasserstoff steht, **dadurch gekennzeichnet, dass** man (2S)-3-Aminopropan-1,2-diol der Formel (II) 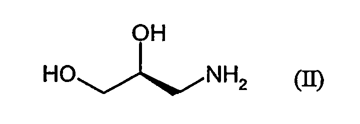 in einem inerten Lösungsmittel in Gegenwart einer Base mit einer Verbindung der Formel (III) 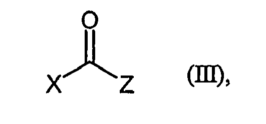 in welcher Z die in Anspruch 1 angegebene Bedeutung aufweist und
X für eine geeignete Abgangsgruppe wie beispielsweise Halogen, bevorzugt für Chlor steht,
zu Verbindungen der Formel (IV) 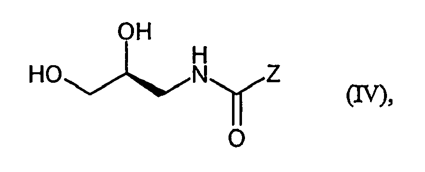 in welcher Z die oben angegebene Bedeutung aufweist,
umsetzt, anschließend mit Hilfe von Bromwasserstoffsäure in Essigsäure, gegebenenfalls unter Zusatz von Essigsäureanhydrid, in Verbindungen der Formel (V) 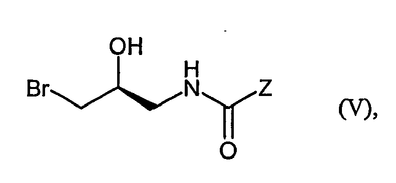 in welcher Z die oben angegebene Bedeutung aufweist,
überführt, diese dann in einem inerten Lösungsmittel in Gegenwart einer Base zu Verbindungen der Formel (VI) 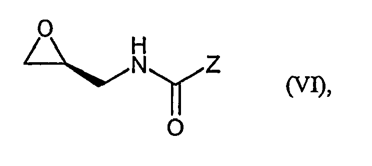 in welcher Z die oben angegebene Bedeutung aufweist,
cyclisiert, nachfolgend in einem inerten Lösungsmittel, gegebenenfalls in Gegenwart einer Protonen- oder Lewis-Säure, mit einer Verbindung der Formel (VII) 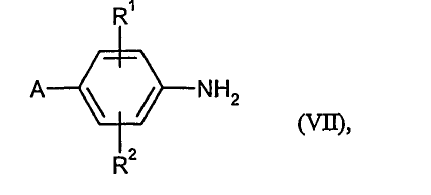 in welcher A, R¹ und R² die in Anspruch 1 angegebenen Bedeutungen aufweisen, zu Verbindungen der Formel (VIII) 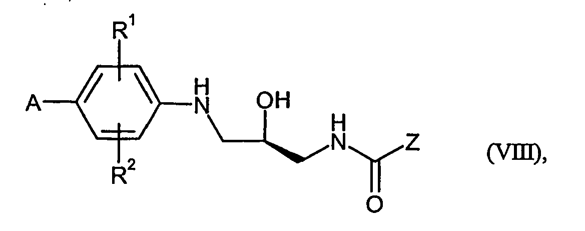 in welcher A, Z, R¹ und R² die oben angegebenen Bedeutungen aufweisen,
umsetzt und diese dann in einem inerten Lösungsmittel mit Bromcyan, gegebenenfalls in Gegenwart einer Säure, zu Verbindungen der Formel (I-A) 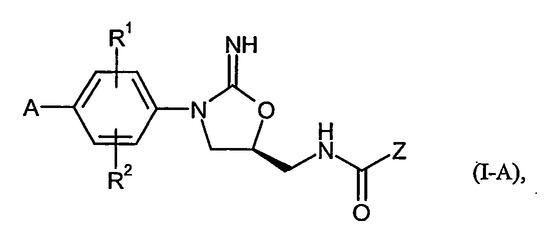 in welcher A, Z, R¹ und R² die oben angegebenen Bedeutungen aufweisen,
reagiert
und die Verbindungen der Formel (I-A) gegebenenfalls mit den entsprechenden (i) Lösungsmitteln und/oder (ii) Basen oder Säuren zu ihren Solvaten, Salzen und/oder Solvaten der Salze umsetzt.

5. Verbindung der Formel (I), wie in Anspruch 1 definiert, zur Behandlung und/oder Prophylaxe von Krankheiten.

6. Verwendung einer Verbindung der Formel (I), wie in Anspruch 1 definiert, zur Herstellung eines Arzneimittels zur Behandlung und/oder Prophylaxe von thromboembolischen Erkrankungen.

7. Verwendung einer Verbindung der Formel (I), wie in Anspruch 1 definiert, zur Verhinderung der Blutkoagulation in vitro.

8. Arzneimittel enthaltend eine Verbindung der Formel (I), wie in Anspruch 1 definiert, in Kombination mit einem inerten, nicht-toxischen, pharmazeutisch geeigneten Hilfsstoff.

9. Arzneimittel enthaltend eine Verbindung der Formel (I), wie in Anspruch 1 definiert, in Kombination mit einem weiteren Wirkstoff.

10. Arzneimittel nach Anspruch 8 oder 9 zur Behandlung und/oder Prophylaxe von thromboembolischen Erkrankungen.

11. Verfahren zur Verhinderung der Blutkoagulation in vitro, **dadurch gekennzeichnet, dass** eine antikoagulatorisch wirksame Menge einer Verbindung der Formel (I), wie in Anspruch 1 definiert, zugegeben wird.

## Claims

1. Compound of the formula (I) 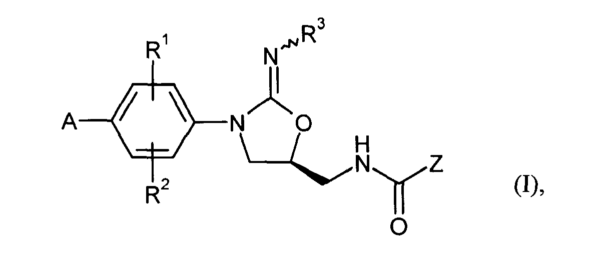 in which
A represents a group of the formula 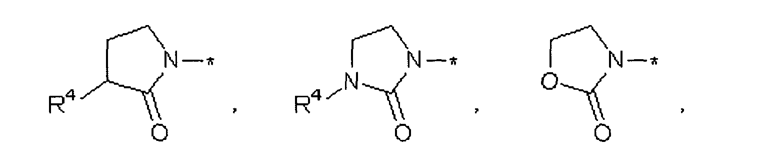 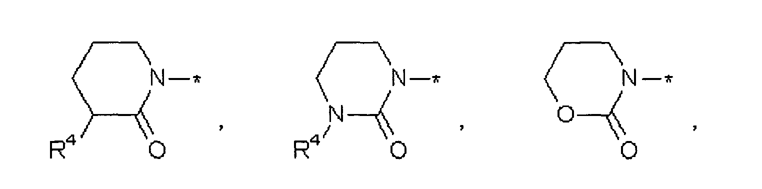 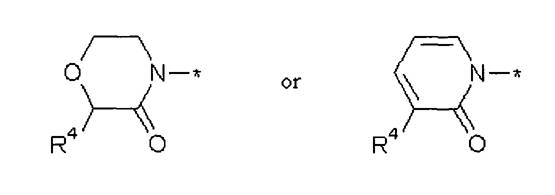 in which
R⁴ represents hydrogen, (C₁-C₆)-alkyl, (C₃-C₇)-cycloalkyl, hydroxy, (C₁-C₆)-alkoxy, amino, mono- or di-(C₁-C₆)-alkylamino, (C₃-C₇)-cycloalkylamino, (C₁-C₆)-alkanoylamino or (C₁-C₆)-alkoxycarbonylamino, where
(C₁-C₆)-alkyl, (C₁-C₆)-alkoxy, mono- and di-(C₁-C₆)-alkylamino for their part may in each case be substituted by hydroxyl, (C₁-C₄)-alkoxy, amino, mono- or di-(C₁-C₄)-alkylamino, (C₃-C₇)-cycloalkylamino or a 4- to 7-membered saturated heterocycle which is attached via a nitrogen atom and which may contain a ring member from the group consisting of N-R⁵ and O, where
R⁵ represents hydrogen or (C₁-C₄)-alkyl,
and * denotes the point of attachment to the phenyl ring,
or
A represents a group of the formula -C(=O)-NR⁶R⁷, where
R⁶ and R⁷ are identical or different and independently of one another are (C₁-C₆)-alkyl or (C₃-C₇)-cycloalkyl
or
R⁶ and R⁷ together with the nitrogen atom to which they are attached form a 4- to 6-membered saturated or partially unsaturated heterocycle which may contain one ring member from the group consisting of N-R⁸ and O and which may be substituted by (C₁-C₆)-alkyl, hydroxy, (C₁-C₆)-alkoxy, oxo, amino, mono- or di-(C₁-C₆)-alkylamino, where
R⁸ represents hydrogen or (C₁-C₆)-alkyl, where for their part all (C₁-C₆)-alkyl groups mentioned may be substituted by hydroxyl, (C₁-C₄)-alkoxy, amino, mono- or di-(C₁-C₄)-alkylamino or (C₃-C₇)-cycloalkylamino,
Z represents phenyl, pyridyl, pyrimidinyl, pyrazinyl, thienyl, furyl or pyrrolyl which may in each case be mono- or disubstituted by identical or different substituents selected from the group consisting of halogen, cyano, (C₁-C₄)-alkyl, which for its part may be substituted by hydroxyl or amino, ethynyl, cyclopropyl and amino,
R¹ and R² are identical or different and independently of one another represent hydrogen, halogen, cyano, (C₁-C₄)-alkyl, cyclopropyl, trifluoromethyl, hydroxyl, (C₁-C₄)-alkoxy, trifluoromethoxy, amino, mono- or di-(C₁-C₄)-alkylamino, where
(C₁-C₄)-alkyl and (C₁-C₄)-alkoxy for their part may in each case be substituted by hydroxyl, (C₁-C₄)-alkoxy, amino, mono- or di-(C₁-C₄)-alkylamino or (C₃-C₇)-cycloalkylamino,
and
R³ represents hydrogen, (C₁-C₆)-alkyl or cyano,
and salts, solvates and solvates of the salts thereof.

2. Compound of the formula (I) according to Claim 1 in which
A represents a group of the formula 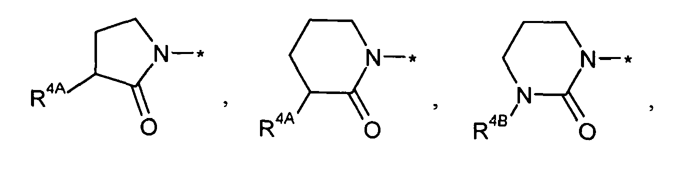 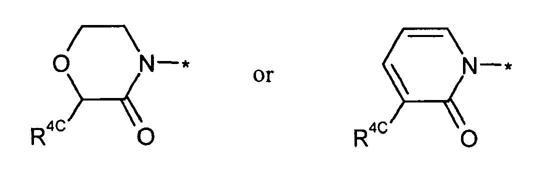 in which
R^{4A} represent hydrogen, hydroxyl, methoxy or amino,
R^{4B} represents methyl or ethyl which may in each case be substituted by hydroxyl, amino, pyrrolidino or cyclopropylamino, or represents amino,
R^{4C} represents hydrogen, methyl or ethyl, where methyl and ethyl may in each case be substituted by hydroxyl, amino, pyrrolidino or cyclopropylamino,
and
* denotes the point of attachment to the phenyl ring,
Z represents a group of the formula 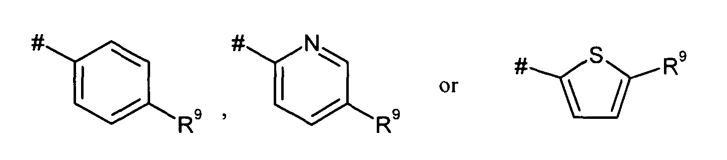 in which
R⁹ represents fluorine, chlorine, methyl or ethynyl
and
# denotes the point of attachment to the carbonyl group,
R¹ represents hydrogen,
R² represents hydrogen, fluorine or methyl,
and
R³ represents hydrogen or (C₁-C₄)-alkyl,
and salts, solvates and solvates of the salts thereof.

3. Compound of the formula (I) according to Claim 1 or 2 in which
A represents a group of the formula 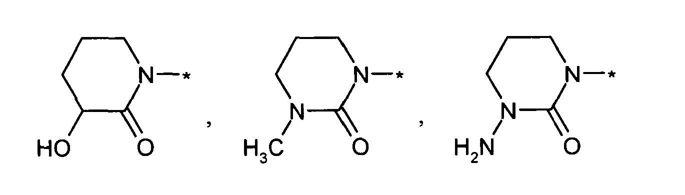 or 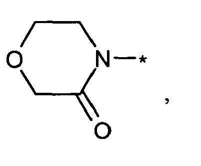 in which * denotes the point of attachment to the phenyl ring,
Z represents a group of the formula 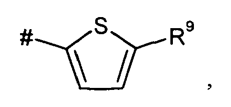 in which
R⁹ represents fluorine, chlorine or methyl
and
# denotes the point of attachment to the carbonyl group,
R¹ represents hydrogen,
R² represents hydrogen, fluorine or methyl,
and
R³ represents hydrogen,
and salts, solvates and solvates of the salts thereof.

4. Process for preparing compounds of the formula (I) as defined in Claim 1 in which R³ represents hydrogen, **characterized in that** (2*S*)-3-aminopropane-1,2-diol of the formula (II) 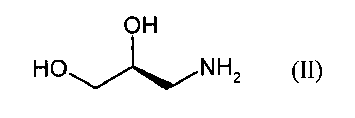 is reacted in an inert solvent in the presence of a base with a compound of the formula (III) 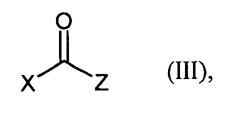 in which Z has the meaning given in Claim 1 and
X represents a suitable leaving group such as, for example, halogen, preferably chlorine,
to give compounds of the formula (IV) 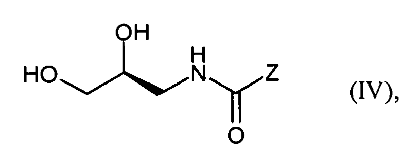 in which Z has the meaning given above,
then converted with the aid of hydrobromic acid in acetic acid, if appropriate with addition of acetic anhydride, into compounds of the formula (V) 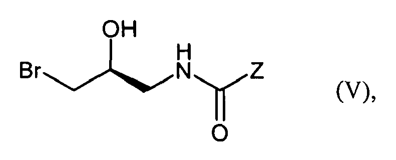 in which Z has the meaning given above,
these are then cyclized in an inert solvent in the presence of a base into compounds of the formula (VI) 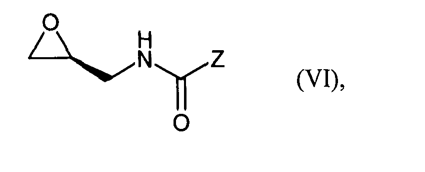 in which Z has the meaning given above,
then reacted in an inert solvent, if appropriate in the presence of a protic acid or Lewis acid, with a compound of the formula (VII) 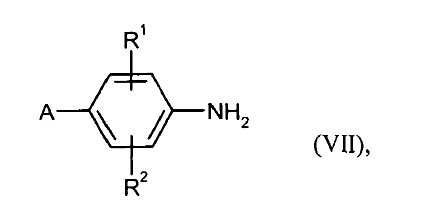 in which A, R¹ and R² have the meanings given in Claim 1, to give compounds of the formula (VIII) 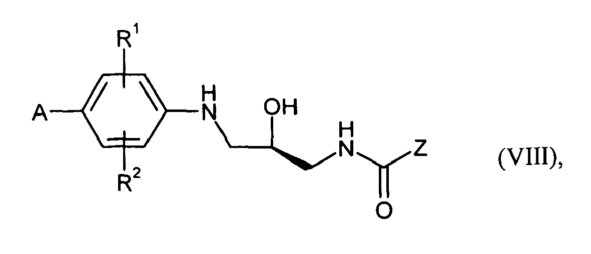 in which A, Z, R¹ and R² have the meanings given above,
and these are then reacted in an inert solvent with cyanogen bromide, if appropriate in the presence of an acid, to give compounds of the formula (I-A) 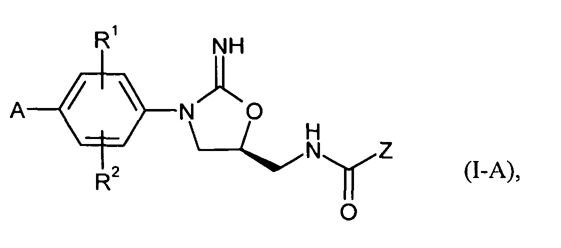 in which A, Z, R¹ and R² have the meanings given above,
and the compounds of the formula (I-A) are, if appropriate, converted with the appropriate (i) solvents and/or (ii) bases or acids into their solvates, salts and/or solvates of the salts.

5. Compound of the formula (I) as defined in Claim 1 for the treatment and/or prophylaxis of diseases.

6. Use of a compound of the formula (I) as defined in Claim 1 for preparing a medicament for the treatment and/or prophylaxis of thromboembolic disorders.

7. Use of a compound of the formula (I) as defined in Claim 1 for preventing blood coagulation in vitro.

8. Medicament, comprising a compound of the formula (I) as defined in Claim 1 in combination with an inert non-toxic, pharmaceutically suitable auxiliary.

9. Medicament, comprising a compound of the formula (I) as defined in Claim 1 in combination with a further active compound.

10. Medicament according to Claim 8 or 9 for the treatment and/or prophylaxis of thromboembolic disorders.

11. Method for prevening blood coagulation in vitro, **characterized in that** an anticoagulatory effective amount of a compound of the formula (I) as defined in Claim 1 is added.

## Revendications

1. Composé de formule (I) 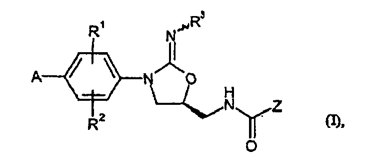 dans laquelle
A représente un groupe de formule 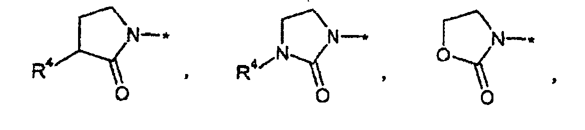 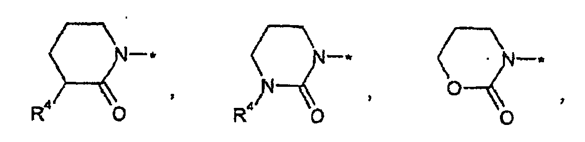 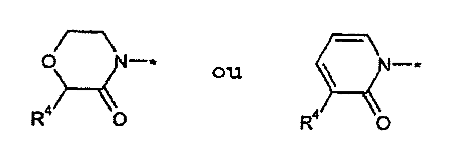 formules dans lesquelles
R⁴ représente un atome d'hydrogène, un groupe alkyle en C₁-C₆, cycloalkyle en C₃-C₇, hydroxy, alcoxy en C₁-C₆, amino, mono- ou dialkyl(C₁-C₆) amino, cycloalkyl(C₃-C₇) amino, alcanoyl(C₁-C₆)amino ou alcoxy (C₁-C₆) carbonylamino,
les groupes alkyle en C₁-C₆, alcoxy en C₁-C₆, mono- et dialkyl(C₁-C₆)amino pouvant pour leur part être substitués chacun par hydroxy, alcoxy en C₁-C₄, amino, mono- ou dialkyl(C₁-C₄) amino, cycloalkyl(C₃-C₇) amino ou par un hétérocycle saturé à 4-7 chaînons lié par un atome d'azote, qui peut contenir un chaînon de cycle choisi dans l'ensemble constitué par N-R⁵ et O, où
R⁵ représente un atome d'hydrogène ou un
groupe alkyle en C₁-C₄,
et * représente la position de liaison avec le cycle phényle,
ou
A représente un groupe de formule -C(=O)-NR⁶R⁷, dans laquelle
R⁶ et R⁷ sont identiques ou différents et
représentent, indépendamment l'un de l'autre, un groupe alkyle en C₁-C₆ ou cycloalkyle en C₃-C₇
ou
R⁶ et R⁷ forment ensemble, avec l'atome d'azote auquel ils sont liés, un hétérocycle à 4-6 chaînons, saturé ou partiellement insaturé, qui peut contenir un chaînon de cycle choisi dans l'ensemble constitué par N-R⁸ et O et être substitué par alkyle en C₁-C₆, hydroxy, alcoxy en C₁-C₆, oxo, amino, mono- ou dialkyl(C₁-C₆)amino, où
R⁸ représente un atome d'hydrogène ou un groupe alkyle en C₁-C₆,
tous les groupes alkyle en C₁-C₆ cités pouvant pour leur part être substitués par hydroxy, alcoxy en C₁-C₄, amino, mono- ou dialkyl(C₁-C₄) amino ou cycloalkyl (C₃-C₇) amino,
Z représente un groupe phényle, pyridyle, pyrimidinyle, pyrazinyle, thiényle, furyle ou pyrrolyle, qui peuvent porter chacun un ou deux substituants, identiques ou différents, choisis dans l'ensemble constitué par des atomes d'halogène et des groupes cyano, alkyle en C₁-C₄, qui peut pour sa part être substitué par hydroxy ou amino, des groupes éthynyle, cyclopropyle et amino,
R¹ et R² sont identiques ou différents et représentent indépendamment l'un de l'autre, un atome d'hydrogène ou d'halogène ou un groupe cyano, alkyle en C₁-C₄, cyclopropyle, trifluorométhyle, hydroxy, alcoxy en C₁-C₄, trifluorométhoxy, amino, mono- ou dialkyl(C₁-C₄)amino,
les groupes alkyle en C₁-C₄ et alcoxy en C₁-C₄, pouvant pour leur part être substitués chacun par hydroxy, alcoxy en C₁-C₄, amino, mono- ou dialkyl(C₁-C₄)amino ou cycloalkyl(C₃-C₇)amino,
et
R³ représente un atome d'hydrogène, un groupe alkyle en C₁-C₆ ou cyano,
ainsi que leurs sels, produits de solvatation et produits de solvatation des sels.

2. Composé de formule (I) selon la revendication 1, dans lequel
A représente un groupe de formule 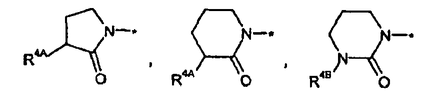 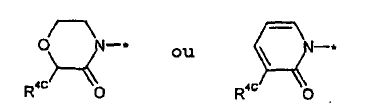
R^{4A} représente un atome d'hydrogène, le groupe hydroxy, méthoxy ou amino,
R^{4B} représente un groupe méthyle ou éthyle, qui peuvent être substitués chacun par hydroxy, amino, pyrrolidino ou cyclopropylamino, ou un groupe amino,
R^{4C} représente un atome d'hydrogène, un groupe méthyle ou éthyle, les groupes méthyle et éthyle pouvant être substitués chacun par hydroxy, amino, pyrrolidino ou cyclopropylamino,
et
* représente la position de liaison avec le cycle phényle,
Z représente un groupe de formule 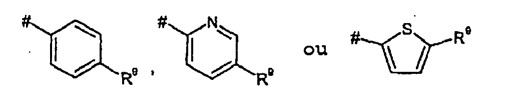 où
R⁹ représente un atome de fluor ou de chlore ou le groupe méthyle ou éthynyle
et
# représente la position de liaison avec le groupe carbonyle,
R¹ représente un atome d'hydrogène,
R² représente un atome d'hydrogène ou de fluor ou le groupe méthyle,
et
R³ représente un atome d'hydrogène ou un groupe alkyle en C₁-C₄,
ainsi que leurs sels, produits de solvatation et produits de solvatation des sels.

3. Composé de formule (I) selon la revendication 1 ou 2, dans lequel
A représente un groupe de formule 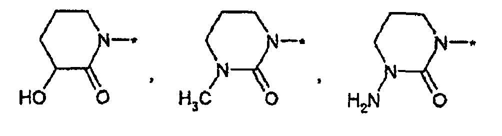 ou 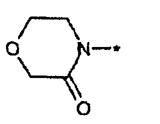 où * représente la position de liaison avec le cycle phényle,
Z représente un groupe de formule 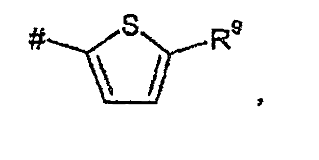
R⁹ représente un atome de fluor ou de chlore ou le groupe méthyle
et
# représente la position de liaison avec le groupe carbonyle,
R¹ représente un atome d'hydrogène,
R² représente un atome d'hydrogène ou de fluor ou le groupe méthyle,
et
R³ représente un atome d'hydrogène,
ainsi que leurs sels, produits de solvatation et produits de solvatation des sels.

4. Procédé pour la préparation de composés de formule (I), tels que définie dans la revendication 1, dans lesquels R³ représente un atome d'hydrogène, **caractérisé en ce qu'**on fait réagir du (2*S*)-3-aminopropane-1,2-diol de formule (II) 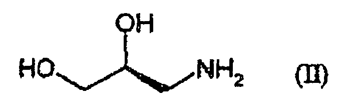 dans un solvant inerte, en présence d'une base, avec un composé de formule (III) 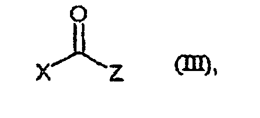 dans laquelle Z a la signification indiquée dans la revendication 1, et
X représente un groupe partant, comme par exemple un atome d'halogène, de préférence de chlore,
pour aboutir à des composés de formule (IV) 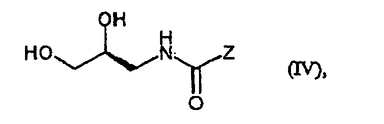 dans laquelle Z a la signification indiquée plus haut,
ensuite on les convertit, à l'aide d'acide bromhydrique dans de l'acide acétique, éventuellement avec addition d'anhydride acétique, en composés de formule (V) 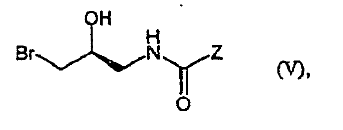 dans laquelle Z a la signification donnée plus haut,
puis on cyclise ces derniers dans un solvant inerte, en présence d'une base, en composés de formule (VI) 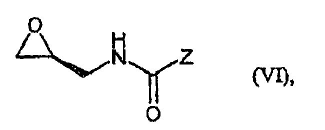 dans laquelle Z a la signification indiquée plus haut,
ensuite on les fait réagir dans un solvant inerte, éventuellement en présence d'un acide protonique ou de Lewis, avec un composé de formule (VII) 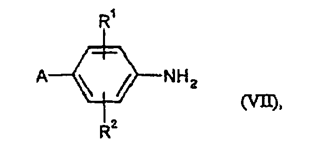 dans laquelle A, R¹ et R² ont les significations indiquées dans la revendication 1,
pour aboutir à des composés de formule (VIII) 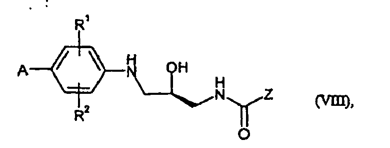 dans laquelle A, Z, R¹ et R² ont les significations indiquées plus haut,
et on fait ensuite réagir ces derniers, dans un solvant inerte, avec du bromure de cyanogène, éventuellement en présence d'un acide, pour aboutir aux composés de formule (I-A) 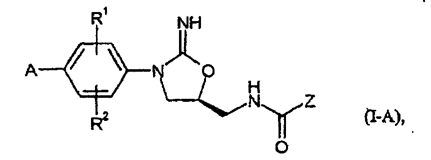 dans laquelle A, Z, R¹ et R² ont les significations indiquées plus haut,
et on convertit les composés de formule (I-A), éventuellement avec (i) les solvants appropriés et/ou (ii) des bases ou des acides convenables, en leurs produits de solvatation, sels et/ou produits de solvatation des sels.

5. Composé de formule (I), telle que définie dans la revendication 1, pour le traitement et/ou la prophylaxie de maladies.

6. Utilisation d'un composé de formule (I), telle que définie dans la revendication 1, pour la fabrication d'un médicament destiné au traitement et/ou à la prophylaxie de maladies thromboemboliques.

7. Utilisation d'un composé de formule (I), telle que définie dans la revendication 1, pour empêcher la coagulation du sang in vitro.

8. Médicament contenant un composé de formule (I), telle que définie dans la revendication 1, en association avec un adjuvant inerte, non toxique, pharmaceutiquement approprié.

9. Médicament contenant un composé de formule (I), telle que définie dans la revendication 1, en association avec une autre substance active.

10. Médicament selon la revendication 8 ou 9, destiné au traitement et/ou à la prophylaxie de maladies thromboemboliques.

11. Procédé pour empêcher la coagulation du sang in vitro, **caractérisé en ce qu'**on ajoute une quantité à activité anticoagulante d'un composé de formule (I), telle que définie dans la revendication 1.
